# EUROPÄISCHE PATENTANMELDUNG

(11) **EP 2 246 007 A1**
(43) Veröffentlichungstag der Anmeldung: **03.11.2010**
(21) Anmeldenummer: 09005875.1
(22) Anmeldetag: 28.04.2009
(51) Int. Cl.: A61C 1/08, A61C 8/00, A61B 17/16, A61B 19/00

(54) **Dentalbohrsystem**

(71) Anmelder: Nanolize GmbH, 69493 Hirschberg (DE)
(72) Erfinder: Witt, Andreas, DE-34305 Niedenstein (DE)
(74) Vertreter: Tergau & Pohl Patentanwälte

(57) **Zusammenfassung**

Ein Dentalbohrsystem (1) mit einem über einen Antriebsschaft (4) angetriebenen Bohrer (2), soll eine sowohl im Hinblick auf die Positionierung als auch im Hinblick auf die Orientierung besonders präzise und zuverlässige Anbringung des Bohrlochs im Kieferknochen ermöglichen. Dazu ist der Bohrer (12) erfindungsgemäß in seinem Schneidbereich (10) in seiner Axialrichtung verschiebbar in einer Führungshülse (18) gelagert, wobei die Führungshülse (18) in ihrem Außendurchmesser an den Innendurchmesser einer Sollbohrung (14) in einer zugeordneten, in den Patientenmund einbringbaren Bohrschablone (12) angepasst ist.

## Beschreibung

Die Erfindung bezieht sich auf ein Dentalbohrsystem mit einem über einen Antriebsschaft angetriebenen Bohrer.

Dentalimplantate sind in vielfältigen Formen bekannt. Sie werden üblicherweise anstelle eines extrahierten oder ausgefallenen Zahnes in den Kieferknochen eingesetzt, um dort nach einer Einheilphase von 3 bis 4 Monaten ein als Zahnersatz dienendes prothetisches Teil oder eine Krone zu halten. Dazu ist ein derartiges Dentalimplantat üblicherweise als geeignet geformter Metallkörper ausgebildet, wobei das Pfostenteil üblicherweise durch Einschrauben an der vorgesehenen Stelle in den Kieferknochen eingesetzt wird. Das Pfostenteil weist dabei in der Regel am apikalen Ende ein zumeist selbstschneidendes Schraubengewinde auf, mit welchem das Pfostenteil in das entsprechend präparierte Implantatbett eingesetzt wird.

Zur Vorbereitung des als Implantatbett vorgesehenen Kieferknochens wird dieser vor der Einbringung des Pfostenteils üblicherweise geeignet vorbehandelt, wobei insbesondere ein geeignet dimensioniertes Bohrloch im Kieferknochen platziert wird, um das eigentliche Einschrauben des Pfostenteils zu erleichtern und die Behinderung beim Einschrauben durch Materialabtrag und dergleichen besonders gering zu halten. Zu diesen oder auch zu anderen Zwecken werden Dentalbohrsysteme mit einem über einen Antriebsschaft angetriebenen Bohrer herangezogen.

Bei der Vorbereitung des Kieferknochens zur Einbringung des Pfostenteils ist eine präzise Anbringung des Bohrlochs im Kieferknochen von besonderer Bedeutung. Dabei besteht üblicherweise einerseits das Bestreben, das Bohrloch im Kieferknochen gerade im Hinblick auf die später vorgesehene Anbringung eines Zahnersatzstücks oder einer Prothese geeignet positioniert anzubringen. Zu diesem Zweck werden üblicherweise so genannte Bohrschablonen verwendet, die außerhalb des Patientenmundes hergestellt werden und derart konfiguriert sind, dass sie an patientenspezifische oder anatomische (?) Merkmale, insbesondere Zahnbestand, Schleimhaut oder Knochen, angepasst und somit in definierter Position in den Patientenmund einbringbar sind oder auf gezielt platzierten Intenplantaten positioniert werden können. In einer derartigen Bohrschablone werden sodann nach erfolgter Diagnostik an den vorgesehenen Stellen geeignete Bohrlöcher angebracht, so dass bei eingesetzter Bohrschablone im Patientenmund für den behandelnden Arzt oder Chirurgen die für das Bohrloch im Kieferknochen vorgesehene Position besonders leicht auffindbar ist.

Im Hinblick auf eine besonders hochwertige therapeutische Versorgung und eine besonders präzise Anfertigung der Zahnersatzstücke oder Prothesen ist darüber hinaus aber auch noch wünschenswert, zusätzlich zu einer korrekten flächigen Positionierung des Bohrlochs auch noch dessen Eindringtiefe und Eindringrichtung im Kieferknochen angepasst und geeignet vorzugeben, so dass Positionierungs- oder Orientierungsfehler bei der Einbringung des Pfostenteils für das Dentalimplantat besonders gering gehalten werden können.

Zur Einhaltung einer extern vorgegebenen Eindringtiefe eines Dentalbohrers in das Zahnfleisch oder den Kieferknochen ist es beispielsweise aus der EP 1 759 654 A1 bekannt, einen Dentalbohrer mit einem Stoppelement zu versehen. Dieses Stoppelement ist geeignet am Bohrschaft fixierbar und liefert damit eine Anschlagsfläche für den Bohrvorgang, so dass die maximale Eindringtiefe des Bohrers über die geeignete feste Verankerung des Stoppelements am Bohrschaft vorgegeben werden kann.

Derartige Dentalbohrer erlauben somit zwar die Voreinstellung der vorgesehenen Eindringtiefe in den Kieferknochen. Dennoch ist bei derartigen Systemen aber eine Vorgabe der Ausrichtung im Kieferknochen nur unzureichend möglich, so dass bei der Vorbereitung des Kieferknochens für die Einbringung des Pfostenteils des Dentalimplantats Positionierungs- und Orientierungsfehler auftreten können.

Der Erfindung liegt daher die Aufgabe zugrunde, ein Dentalbohrsystem mit einem über einen Antriebsschaft angetriebenen Bohrer anzugeben, mit dem eine sowohl im Hinblick auf die Positionierung als auch im Hinblick auf die Orientierung besonders präzise und zuverlässige Anbringung des Bohrlochs im Kieferknochen ermöglicht ist.

Diese Aufgabe wird erfindungsgemäß gelöst, indem der Bohrer in seinem Schneidbereich in seiner Axialrichtung verschiebbar in einer Führungshülse gelagert ist, wobei die Führungshülse in ihrem Außendurchmesser an den Innendurchmesser einer Sollbohrung in einer zugeordneten, in den Patientenmund einbringbaren Bohrschablone angepasst ist.

Die Erfindung geht dabei von der Überlegung aus, dass für eine hohe Positioniergenauigkeit des jeweiligen Bohrlochs an der Oberfläche des Kieferknochens grundsätzlich auf die zu diesem Zweck bewährten, im Patientenmund einbringbaren Bohrschablonen mit den darin eingebrachten Sollbohrungen zurückgegriffen werden sollte. Um über die Positioniergenauigkeit hinaus aber auch die Ausrichtung des Bohrlochs im Kieferknochen mit besonders hoher Genauigkeit sicherstellen und damit Orientierungsfehler beim Einbringen des Bohrlochs in den Kieferknochen besonders weitgehend vermeiden zu können, sollte die Bohrschablone über die Vorgabe einer Positionierungsbedingung im Patientenmund hinaus auch zur Vorgabe einer Richtungsinformation bei der Anbringung des Bohrlochs nutzbar gemacht werden.

Um dies zu ermöglichen, ist vorgesehen, auf geeignete Weise eine Führung in Längsrichtung an der Sollbohrung der Bohrschablone auf den Schneidbereich des Bohrers zu übertragen. Um hierbei Verklemmungen, Beschädigungen oder dergleichen besonders zuverlässig zu vermeiden, ist vorgesehen, die Richtungsinformation von der Sollbohrung in der Bohrschablone zunächst auf eine Führungshülse für den Schneidbereich des Bohrers zu übertragen, die ihrerseits durch geeignete Lagerung am Schneidbereich und/oder Schaft des Bohrers im in die Sollbohrung eingesteckten Zustand eine korrekte Ausrichtung des Bohrers an sich sicherstellt. Um eine derartige Übertragung der Richtungsinformation von der Sollbohrung auf die Führungshülse zu ermöglichen, sollte die Führungshülse im Hinblick auf die Sollbohrung, insbesondere durch geeignete Wahl ihres Außendurchmessers, geeignet dimensioniert und an die Sollbohrung angepasst sein.

Um einen Materialaustrag aus dem Bohrloch in den umgebenden Mundraum oder in die hierdurch entstehende Wunde und damit auch das Infektionsrisiko besonders gering zu halten, umschließt die Führungshülse vorteilhafterweise den Schneidbereich des Bohrers an seinem vom Bohrende abgewandten Ende kapselartig. Dabei kann die Führungshülse insbesondere den zylindrischen Bohrschaft oberhalb des Schneidbereichs des Bohrers geeignet umfassen, wobei die Durchführung des Bohrerschafts durch diesen Deckelbereich der Führungshülse eine gleitende Lagerung der Führungshülse am Bohrerschaft bedingt.

In weiterer vorteilhafter Ausgestaltung weist dabei die Führungshülse im Zentralbereich der Kapselung einen im Vergleich zum Außendurchmesser des Schneidbereichs des Bohrers mindestens um 0,01mm, vorzugsweise um etwa 0,05 bis 0,1mm, größeren Innendurchmesser auf. Dadurch ist sichergestellt, dass der Schneidbereich des Bohrers im Inneren der Führungshülse frei um seine Längsachse rotieren kann, so dass zumindest in diesem Raumbereich keine den Bohrvorgang beeinträchtigenden Reibungs- oder Verlusteffekte auftreten. Die Lagerung und/oder Führung des Bohrers in der Führungshülse tritt dabei im Wesentlichen nur an den Enden der Kapselung, also einerseits im Bereich des Durchtritts des Antriebsschafts durch die Deckelplatte der Führungshülse und andererseits im Bereich des Austrittsschneidbereich des Bohrers zum unteren Ende der Führungshülse, auf, so dass die insgesamt auftretende Reibung besonders gering gehalten und ein besonders ruhiger verlustarmer Lauf des Bohrers gewährleistet ist.

In weiterer vorteilhafter Ausgestaltung ist dabei der Antriebsschaft des Bohrers durch die Deckelplatte der Führungshülse geführt und im Bereich dieser Durchführung konisch ausgestaltet. Durch die konische Ausgestaltung des Antriebsschafts in diesem Bereich kann die Führungshülse vor der Einbringung in die zugeordnete Sollbohrung in der Bohrschablone am Bohrerschaft verklemmt und damit vorübergehend fixiert werden, so dass beim Einbringen oder Einfädeln der Führungshülse in die Sollbohrung ein Verrutschen der Führungshülse nach oben hin erschwert oder sogar ganz vermieden ist.

Um zusätzlich zur korrekten Positionierung und Ausrichtung des Bohrlochs im Kieferknochen auch noch dessen Eindringtiefe in den Kieferknochen besonders zuverlässig vorgeben zu können, weist die Führungshülse in besonders vorteilhafter Ausgestaltung an ihrem vom Bohrende abgewandten Ende eine Anschlagsfläche für ein Stoppelement auf. Das Stoppelement kann dabei in besonders vorteilhafter Weiterbildung als auf ein Außengewinde des Antriebsschafts aufschraubbare Schraubenmutter ausgebildet sein. Damit kann die vorgesehene Eindringtiefe des Bohrers durch eine geeignete Voreinstellung der Schraubenmutter am Bohrerschaft vorgenommen werden, so dass mit besonders einfachen Mitteln die Einhaltung der korrekt bemessenen Eindringtiefe sichergestellt ist.

Um dabei auch bei nur begrenzten räumlichen Möglichkeiten, insbesondere im Hinblick auf die insgesamt vorgesehene Baulänge des Systems, einen besonders großen Parameterbereich verschiedenartiger Eindringtiefen zuverlässig abdecken zu können, ist in vorteilhafter Weiterbildung zwischen das Stoppelement und die Anschlagsfläche der Führungshülse ein Abstandshalter einbringbar. Nach Art einer Grobeinstellung kann somit durch die Wahl eines Abstandshalters in geeigneter Länge, beispielsweise aus einem bereitgestellten Set verschiedenartiger Abstandshalter, eine grob bemessene Eindringtiefe vorausgewählt und anschließend durch korrekte Positionierung des Stoppelements oder der Schraubenmutter fein eingestellt werden. Die in der Art eines Satzes bereitgestellten verschiedenartigen Abstandshalter können dabei insbesondere in ihrer Dimensionierung an die anderen Komponenten angepasst sein und aus einem sterilisierbaren Kunststoff, beispielsweise PEEK oder aus Titan oder Edelstahl gefertigt. Alternativ oder zusätzlich kann der Antriebsschaft des Bohrers, vorzugsweise im Bereich zwischen dem Stoppelement und der Anschlagfläche der Führungshülse mit einer Anzahl optischer Markierugen zur Positionsbestimmung versehen sein, die beispielsweise in der Art einer Skala eine optische Positionierhilfe oder Bohrtiefenkontrolle ermöglichen. Bei der Dimensionierung aller Komponenten ist zudem vorteilhafterweise berücksichtigt, dass der maximale Außendurchmesser durch einen an der Führungshülse angeordneten, als Gegenanschlag für die Bohrschablone dienenden umlaufenden Kragen oder Ringwulst vorgegeben ist.

Für eine besonders zuverlässige Führung des Bohrers innerhalb der Führungshülse ist der Schneidbereich des Bohrers vorteilhafterweise im Übergangsbereich zum Antriebsschaft, also insbesondere innerhalb der Führungshülse, mit einer umlaufenden Verdickung versehen, die in ihrem Außendurchmesser an den Innendurchmesser der Führungshülse angepasst ist. Die Führung des Bohrers innerhalb der Führungshülse erfolgt damit vorzugsweise einerseits über die Durchführung im Deckelbereich und andererseits über die im Kontakt zur Innenwand der Führungshülse geführte Verdickung oberhalb des Schneidbereichs des Bohrers.

Für eine besonders zuverlässige Positionierung und Ausrichtung der Führungshülse in der Bohrschablone weist diese vorzugsweise eine geeignet gewählte Mindestdicke auf, die im Hinblick auf die auftretenden Kräfte und Momente eine Verkippung des in der Führungshülse geführten Bohrschafts relativ zur Bohrschablone mit hinreichender Sicherheit ausschließt. Dazu weist im Hinblick auf die üblicherweise zu berücksichtigenden Größen im Dentalbereich die Bohrschablone vorteilhafterweise im Bereich der Sollbohrung eine Dicke von mindestens 2,5 mm auf.

Grundsätzlich ist die Sollbohrung an sich bereits für eine zuverlässige Aufnahme und Fixierung der Führungshülse in der Bohrschablone geeignet. Um die Aufnahme und Fixierung aber noch weiter zu verbessern, ist die Sollbohrung vorteilhafterweise mit einem Führungsring zur Aufnahme der Führungshülse versehen. Der Führungsring kann dabei insbesondere aus Metall, beispielsweise aus Titan, oder aus einem geeignet gewählten Kunststoff, gebildet sein, so dass bei gering gehaltenem Infektionsrisiko eine mechanisch besonders stabile und zuverlässige Führung und Ausrichtung der angebrachten Führungshülse sichergestellt ist. Zur Erleichterung des Einbringens der Führungshülse in den Führungsring ist dieser in weiterer vorteilhafter Ausgestaltung mit einer Senkung versehen. Diese erleichtert das Einfädeln oder Einbringen der Führungshülse insbesondere dann, wenn diese im Bereich ihres freien Endes mit einer konischen Spitze versehen ist.

Die mit der Erfindung erzielten Vorteile bestehen insbesondere darin, dass durch die axial verschiebliche Lagerung des Bohrers in einer ihn umgebenden Führungshülse diese zuverlässig, präzise und insbesondere unter Einhaltung einer in der Sollbohrung der Bohrschablone vorgegebenen Richtung in die Sollbohrung eingesteckt werden kann. Damit ist auf besonders einfache Weise die Vorgabe einer exakten Positionierung und Ausrichtung des anzubringenden Bohrlochs durch entsprechende Vorfertigung der Sollbohrung, beispielsweise im Herstellerlabor, möglich. Aufwändige Justier- oder Positionierungsarbeiten während der Behandlung, also im Patientenmund, können damit vermieden oder zumindest gering gehalten werden. Zur zuverlässigen Einhaltung einer vorgegebenen Bohrtiefe kann zudem auf das Stoppelement zurückgegriffen werden, das über den Anschlag an der Führungshülse oder dem dazwischengesteckten Abstandshalter das Ausfahren des Schneidbereichs des Bohrers aus der Führungshülse zu einem Maximalwert begrenzt. Der Anschlag erfolgt dabei im Gegensatz zu herkömmlichen Dentalbohrern mit Stoppelement, bei denen das Stoppelement üblicherweise auf Mundgewebe oder dergleichen trifft und dadurch ein weiteres Eindringen des Bohrers begrenzt, durch Kontakt des Stoppelements mit Führungshülse oder Abstandshalter, so dass keine zusätzliche Belastung des menschlichen Gewebes durch das Stoppelement auftritt.

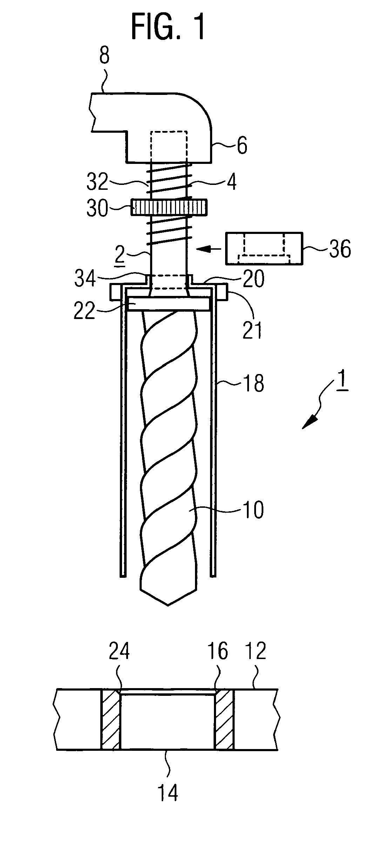

Ein Ausführungsbeispiel der Erfindung wird anhand einer Zeichnung näher erläutert. Darin zeigen die Fig. 1 bis 3 jeweils ein Dentalbohrsystem in unterschiedlichem Betriebszustand.

Gleiche Teile sind in allen Figuren mit denselben Bezugszeichen versehen.

Das Dentalbohrsystem 1 weist einen Bohrer 2 auf, der über einen Antriebsschaft 4, gegebenenfalls über ein Umlenkgelenk 6 oder dergleichen, mit einer Antriebswelle 8 und über diese mit einem nicht näher dargestellten Antriebsmotor verbunden ist. Am bohrseitigen Ende ist an den Antriebsschaft 4 des Bohrers 2 ein Schneidbereich 10 angeformt, über den ein Bohrloch oder Bohrkanal an einer behandlungsbedürftigen Stelle im Patientenmund, insbesondere in einem Kieferknochen, einbringbar ist.

Das Dentalbohrsystem 1 ist für eine hochgenaue und Zeit sparende Anbringung behandlungsspezifischer Bohrlöcher im Kieferknochen oder im Patientenmund ausgelegt, wobei die eigentliche Behandlungsdauer im Patientenmund besonders kurz gehalten werden soll. Dazu ist vorgesehen, anhand vorheriger Diagnoseergebnisse den therapeutischen Behandlungsweg im Vorfeld des Eingriffs vor insbesondere im Hinblick auf ein zu verwendendes Dentalimplantat, eine Zahnprothese oder dergleichen, genau festzulegen. Bereits im Labor, also vor der eigentlichen Behandlung, ist sodann die Herstellung einer so genannten, in den Figuren lediglich auszugsweise dargestellten Bohrschablone 12 vorgesehen, die im Hinblick auf die individuelle Form innerhalb des Patientenmunds passgenau in diesen einbringbar ist. In der Bohrschablone 12 sind dem therapeutischen Bedürfnis entsprechend eine Anzahl von Sollbohrungen 14 angebracht, von denen in den Figuren jeweils lediglich eine zu sehen ist. Die Sollbohrungen sind dabei aufgrund der diagnostischen Erkenntnisse hinsichtlich ihrer Positionierung und Ausrichtung in der Bohrschablone 12 möglichst exakt an das gewünschte Behandlungsergebnis angepasst. Bei Einbringen der Bohrschablone 12 in den Patientenmund kann somit anhand der in dieser enthaltenen Sollbohrungen 14 eine exakte Positionierung und auch Ausrichtung des Bohrers 2 zur Erzeugung der Bohrlöcher erfolgen, wobei im Patientenmund der Bohrer 2 lediglich geeignet durch die Sollbohrungen 14 hindurchgeführt werden muss.

Eine vergleichsweise exakte Positionierung ist dabei bereits durch geeignete Anbringung und Dimensionierung der Sollbohrungen 14 erreichbar. Um darüber hinaus aber auch bereits in der Bohrschablone 12 eine vergleichsweise exakte Ausrichtung des Bohrlochs innerhalb des Kieferknochens, ausgehend von der Eindringstelle des Bohrers in den Kieferknochen, festlegen zu können, ist das Dentalsystem 1 in seiner Gesamtheit für die mögliche Übertragung einer bereits in der Sollbohrung 14 enthaltenen Ausrichtung des Bohrkanals auf den in die Sollbohrung 14 einzusteckenden Bohrer 2 ausgelegt. Dazu weist die Bohrschablone im Ausführungsbeispiel einerseits eine Dicke von 2,5 mm oder mehr auf, so dass die Sollbohrung 14 in ihrer Innenkontur einen Kanal mit einer Ausrichtung und damit einer Vorzugsrichtung definiert, wobei die Sollbohrung 14 derart ausgerichtet ist, dass die durch sie gebildete Längsrichtung mit der vorgesehenen Ausrichtung des einzubringenden Bohrlochs korrespondiert. Des Weiteren ist die Sollbohrung 14 im Ausführungsbeispiel mit einem

Führungsring 16 aus vergleichsweise hartem und belastbarem Material, insbesondere Titan, versehen, der in die Bohrschablone 12 eingebracht ist und die eigentliche Sollbohrung 14 bildet. Durch den Führungsring 16 ist die Sollbohrung 14 somit als mechanisch vergleichsweise belastbarer Kanal ausgestaltet.

Zur geeigneten Übertragung der durch diesen Kanal gegebenen, bereits im Laborumfeld in die Bohrschablone 12 einbringbaren Ausrichtung auf eine geeignete Ausrichtung des Bohrers 2 beim eigentlichen Bohrvorgang umfasst das Dentalbohrsystem 1 eine Führungshülse 18 für den Bohrer 2. Die

Führungshülse 18 ist in die Sollbohrung 14 einsteckbar und in ihrem Außendurchmesser derart an den Innendurchmesser der Sollbohrung 14 angepasst, dass bei möglichst gering gehaltenem Spiel ein Einschieben der Führungshülse 18 in die Sollbohrung 14 gerade noch weitgehend ohne Verklemmung oder dergleichen möglich ist. Durch das Zusammenwirken von Sollbohrung 14 und daran angepasster Führungshülse 18 ist somit eine Übertragung der bohrseitig in die Sollbohrung 14 eingebrachten Längsrichtung auf die Ausrichtung der Führungshülse 18 möglich, nachdem diese in die Sollbohrung 14 eingesteckt wurde.

Der Bohrer 2 ist in seinem Schneidbereich 10 in der Führungshülse 18 verschiebbar gelagert, so dass sich die Ausrichtung der Längsrichtung der Führungshülse 18 durch die geeignete Lagerung entsprechend auf die Ausrichtung des in dieser verschiebbar gelagerten Bohrers 2 überträgt. Somit ist durch die geeignete Ausrichtung der Sollbohrung 14 im Endeffekt eine geeignete Ausrichtung des Bohrers 2 beim in die Sollbohrung eingesteckten Zustand ermöglicht, so dass Justierarbeiten für die Anbringung des Bohrlochs laborseitig, also außerhalb des Patientenmunds, vorgenommen werden können und nach dem Einlegen der Bohrschablone 12 in den Patientenmund und Einstecken der Führungshülse 18 selbsttätig die Anbringung des Bohrlochs im Kieferknochen in der gewünschten vorgegebenen Ausrichtung erfolgen kann.

Die Führungshülse 18 umschließt dabei ausschnittsweise den Schneidbereich 10 des Bohrers 2 kapselartig, wobei der Antriebsschaft 4 des Bohrers 2 durch einen Deckelbereich 20 der Führungshülse 18 hindurchgeführt ist. Im Ausführungsbeispiel ist die Führungshülse an ihrem dem Bohrende zugewandten Ende offen gehalten, so dass ein ungehinderter Austritt des Schneidbereiches 10 sichergestellt ist. Alternativ könnte die Führungshülse 18 aber auch konisch zugespitzt ausgeführt sein, um die kugelartige Ausführung noch weiter zu verstärken.

Im Bereich der Deckelplatte 20 weist die Führungshülse 18 einen umlaufenden Kragen 21 oder Ringwulst auf, der bei in die Sollbohrung 14 eingesteckter Führungshülse 18 auf der Bohrschablone 12 aufliegt und amit zur Abstützung des Bohres 2 auf der Bohrschablone 12 dient. Zur zusätzlichen Führung und Verbesserung der mechanischen Stabilität ist der Schneidbereich 10 des Bohrers 2 weiter in seinem Übergangsbereich zum Antriebsschaft 4 mit einer umlaufenden Verdickung 22 versehen, die in ihrem Außendurchmesser an den Innendurchmesser der Führungshülse 18 angepasst ist. Der Bohrer 2 steht somit im Wesentlichen lediglich in zwei Bereichen in mechanischem Kontakt mit der Führungshülse 18, nämlich im Bereich der Durchführung des Antriebsschafts 4 durch den Deckel 20 und im Bereich der umlaufenden Verdickung 22. Damit ist der mechanische Kontakt zwischen Bohrer 2 und Führungshülse 18 auf eine vergleichsweise geringe Gesamt-Kontaktfläche reduziert, so dass der Bohrer 2 innerhalb der Führungshülse 18 besonders reibungsarm und verlustarm sowie mit hoher Laufruhe geführt ist. Im Zentralbereich der durch die Führungshülse 18 gebildeten Kapselung ist hingegen ein freier Luftspalt zwischen Außendurchmesser des Schneidbereichs 10 und dem Innendurchmesser der Führungshülse 18 vorgesehen, so dass auch bei innerhalb der Führungshülse 18 rotierendem Bohrer 2 in diesen Bereichen keine Reibungsverluste, Beeinträchtigungen der Laufruhe, Verklemmungen oder sonstigen unerwünschten Beeinträchtigungen des Bohrerlaufs auftreten.

Der Führungsring 16 ist im Übrigen mit einer Senkung 24 versehen, die das Einbringen oder Einfädeln der Führungshülse 18 in die Sollbohrung 14 besonders erleichtert.

Durch die genannte Ausgestaltung ist mit besonders einfachen Mitteln eine zuverlässige Positionierung und auch Ausrichtung des Bohrers 2 unter Rückgriff auf die Bohrschablone 12 im Patientenmund ermöglicht. Um darüber hinaus laborseitig auch die gewünschte Bohrlochtiefe bereits vorwählen oder voreinstellen zu können, ist das Dentalbohrsystem 1 zudem noch mit einem Stoppelement 30 zur Begrenzung der Eindringtiefe des Bohrers 2 in den Kieferknochen auf einen Maximalwert vorgesehen. Das Stoppelement 30 ist dabei als auf einem am Antriebsschaft 4 des Bohrers 2 angebrachten Außengewinde 32 laufende Schraubenmutter ausgebildet. Durch geeignete Verdrehung dieser Schraubenmutter, die im Ausführungsbeispiel mit Rändelrand versehen ist, kann diese somit in ihrer Höhe geeignet auf dem Antriebsschaft 4 positioniert werden.

Beim eigentlichen Bohrvorgang, bei dem der Bohrer 2 relativ zur

Führungshülse 18 aus dieser heraus in den Kieferknochen hinein verschoben wird, tritt das als Schraubenmutter ausgebildete Stoppelement 30 somit bei Erreichen der vorgegebenen Maximaltiefe mechanisch in Kontakt mit einer zugeordneten Anschlagsfläche der Führungshülse 18, nämlich entweder die Außenfläche des Deckels 20 oder einen angeformten, den Antriebsschaft 4 umschließenden umlaufenden Kragen 34. Durch den dabei erfolgenden mechanischen Anschlag des Stoppelements 30 an die jeweilige Anschlagsfläche ist ein weiteres Verschieben des Bohrers 2 in Längsrichtung relativ zur Führungshülse 18 nicht mehr möglich, so dass der Bohrer 2 seine maximale Eindringtiefe in den Kieferknochen erreicht hat. Bedarfsweise kann dieses System noch ergänzt sein durch einen Abstandshalter 36, der - beispielsweise ausgestaltet in der Art eines Kreisringsegments - zwischen das Stoppelement 30 und die Führungshülse 18 eingeschoben werden kann und dabei bereits einen früheren Anschlag des Stoppelements 30 bewirkt. Durch geeignete Auswahl eines Abstandshalters 36, beispielsweise aus einem ganzen Set bereitgehaltener Abstandshalter jeweils unterschiedlicher Dicke, kann dabei bereits in der Art einer Grobjustierung die vorgesehene Eindringtiefe voreingestellt und anschließend durch korrekte Positionierung der Schraubenmutter oder des Stoppelements 30 feineingestellt werden.

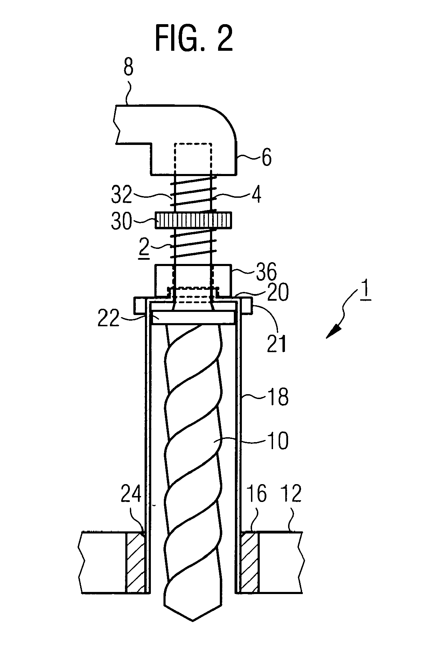

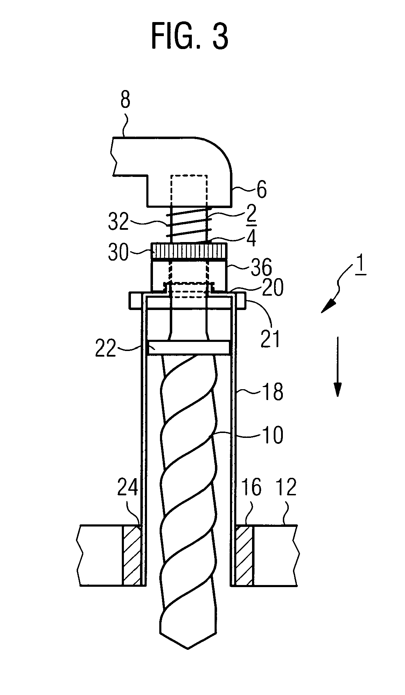

In den Fig. 1 bis 3 ist das Dentalbohrsystem 1 jeweils in unterschiedlichen Betriebszuständen gezeigt. In Fig. 1 ist das Dentalbohrsystem zunächst komponentenweise dargestellt, wobei die Führungshülse 18 noch nicht in die Sollbohrung 14 eingesteckt und der Abstandshalter 36 noch nicht angebracht ist. In Fig. 2 hingegen ist eine Situation vor dem eigentlichen Beginn des Bohrvorgangs dargestellt, bei der die Führungshülse 18 bereits in die Sollbohrung 14 eingesteckt und der Bohrer 2 damit bereits korrekt im Patientenmund positioniert und in seiner Längsrichtung auch korrekt ausgerichtet ist. In Fig. 3 ist das Dentalbohrsystem 1 hingegen in der Situation nach Beendigung des Bohrvorgangs dargestellt, bei der das Stoppelement 30 an die Anschlagsfläche des Abstandshalters 36 angeschlagen und damit der Bohrer 2 bis hin zur maximalen vorgesehenen Eindringtiefe aus der Führungshülse 18 ausgefahren ist.

### Bezugszeichenliste

- 1: Dentalbohrsystem
- 2: Bohrer
- 4: Antriebsschaft
- 6: Umlenkgelenk
- 8: Antriebswelle
- 10: Schneidbereich
- 12: Bohrschablone
- 14: Sollbohrungen
- 16: Führungsring
- 18: Führungshülse
- 20: Deckel
- 21: Kragen
- 22: Verdickung
- 24: Senkung
- 30: Stoppelement
- 32: Gewinde
- 34: Kragen
- 36: Abstandshalter

## Patentansprüche

1. Dentalbohrsystem (1) mit einem über einen Antriebsschaft (4) angetriebenen Bohrer (2), der in seinem Schneidbereich (10) in seiner Axialrichtung verschiebbar in einer Führungshülse (18) gelagert ist, wobei die Führungshülse (18) in ihrem Außendurchmesser an den Innendurchmesser einer Sollbohrung (14) in einer zugeordneten, in den Patientenmund einbringbaren Bohrschablone (12) angepasst ist.

2. Dentalbohrsystem (1) nach Anspruch 1, dessen Führungshülse (18) den Schneidbereich (10) des Bohrers (2) an seinem vom Bohrende (21) abgewandten Ende kapselartig umschließt.

3. Dentalbohrsystem (1) nach Anspruch 2, dessen Führungshülse (18) im Zentralbereich der Kapselung einen im Vergleich zum Außendurchmesser des Schneidbereichs mindestens etwa 0,01 mm größeren Innendurchmesser aufweist.

4. Dentalbohrsystem (1) nach einem der Ansprüche 1 bis 3, bei dem der Antriebsschaft (4) des Bohrers (2) durch eine Deckelplatte (20) der Führungshülse (18) geführt und im Bereich dieser Durchführung konisch ausgestaltet ist.

5. Dentalbohrsystem (1) nach einem der Ansprüche 1 bis 4, dessen Führungshülse (18) an ihrem vom Bohrende abgewandten Ende eine Anschlagsfläche für ein Stoppelement (30) aufweist.

6. Dentalbohrsystem (1) nach Anspruch 6, dessen Stoppelement (30) als auf ein Außengewinde (32) des Antriebsschafts (4) aufschraubbare Schraubenmutter ausgebildet ist.

7. Dentalbohrsystem (1) nach Anspruch 6 oder 7, bei dem zwischen das Stoppelement (30) und die Anschlagsfläche der Führungshülse (18) ein Abstandshalter (36) einbringbar ist.

8. Dentalbohrsystem (1) nach einem der Ansprüche 1 bis 8, bei dem der Schneidbereich (10) des Bohrers (2) im Übergangsbereich zum Antriebsschaft (4) mit einer umlaufenden Verdickung (22) versehen ist, die in ihrem Außendurchmesser an den Innendurchmesser der Führungshülse (18) angepasst ist.

9. Dentalbohrsystem (1) nach einem der Ansprüche 1 bis 9, dessen Bohrschablone (12) im Bereich der Sollbohrung (14) eine Dicke von mindestens 2,5 mm aufweist.

10. Dentalbohrsystem (1) nach einem der Ansprüche 1 bis 10, dessen Bohrschablone (12) im Bereich der Sollbohrung (14) einen Führungsring (16) zur Aufnahme der Führungshülse (18) aufweist.

11. Dentalbohrsystem (1) nach Anspruch 11, bei dem der Führungsring (16) mit einer Senkung (24) versehen ist.
